# EUROPEAN PATENT SPECIFICATION

(11) **EP 2 359 817 B1**
(45) Date of publication and mention of the grant of the patent: **10.01.2018**
(21) Application number: 11165235.0
(22) Date of filing: 29.03.2004
(51) Int. Cl.: A61K 9/50

(54) **Solid oral dosage form containing seamless microcapsules**
Solide orale Dosierform mit nahtlosen Mikrokapseln
Forme posologique solide s'administrant par voie orale qui contient des microcapsules sans soudure

(30) Priority: 28.03.2003 US 458599 P
(43) Date of publication of application: 24.08.2011
(62) Divisional of application: 04724095.7
(73) Proprietor: Sigmoid Pharma Limited, Dublin 9 (IE)
(72) Inventor: Moodley, Joey, Athlone, County Westmeath (IE); Coulter, Ivan, Dublin 6 (IE)
(74) Representative: HGF Limited

(56) References cited:
- EP-A1- 0 778 083
- EP-A1- 1 184 038
- JP-A- 59 088 420
- US-A- 4 597 959

## Description

### Field Of The Invention

The present invention relates to a solid oral dosage form comprising a multiplicity of seamless microcapsules containing a pharmaceutically active ingredient solubilised or dispersed in a pharmaceutically acceptable solvent or liquid phase.

### Background

Drugs for use in therapy and prophylaxis of various medical conditions have varying solubility characteristics ranging from insoluble to lipid soluble and water soluble with varying pH sensitivities. This variation in solubility can affect the therapeutic effectiveness of drugs. Drug dissolution is a prerequisite to drug absorption. Except in very special cases, drugs cannot be absorbed until they are solubilised. If a drug is already in solution at the time of administration, its absorption across the gastrointestinal tract and hence its bioavailability is rapid resulting in a fast therapeutic effect. Rapid or instantaneous bioavailability is characterised by significant blood levels within about 10 to 60 minutes after administration of the drug.

Drugs that are poorly water soluble and/or sensitive to pH must be formulated in a way that improves their solubility and hence their bioavailability. In general, a drug that is in solution or suspension when administered by the oral route is rapidly, and frequently instantaneously, absorbed from the gastrointestinal tract resulting in a fast therapeutic action. However, in many cases, it is desirable to control the rate of drug absorption following oral administration in order to achieve the desired plasma profile or prolongation of action of the drug.

Numerous processes and formulations exist to enhance the solubility of poorly soluble drug compounds. Solubilisation of such drugs in solvents, oils, emulsions and microemulsions are well known to those skilled in the art and have been used to deliver such drugs orally. Such formulations are then encapsulated in soft gelatin capsules for oral administration. Soft gelatin encapsulation is a specialised process, whereby the end products, soft gelatin capsules do not lend themselves easily to further processing such as the addition of delayed or sustained release coatings. Therefore such dosage forms are particularly suited for instantaneous delivery of the encapsulated active drugs.

Particle size reduction processes such as milling, micronisation and spray drying are also well known to increase the surface area and hence solubilisation rates of poorly soluble drugs. U.S. Pat. No. 5,510,118 and US Pat. No. 5,145,684 teaches the milling of such drugs in pharmaceutically acceptable solvents to produce nanosuspensions of the drugs with the resulting enhanced solubility of the drug. The production of nanosuspension/nanodispersion of poorly soluble drugs can be performed by using a double nozzle spray drying process. These nanosuspensions or nanodispersions result in increased dissolution rates of the active and increased absorption and bioavailability. However for administration as a solid oral dosage form with controlled and/or sustained release characteristics, the nanodispersions or nanosuspensions are subsequently dried to produce free flowing powder for further processing into a solid oral dosage form such as tablets, minitablets which can then be coated with a controlled or sustained release polymers to control the delivery rate of the active. Recovery of the nanoparticle size distribution on dissolution of these dosage forms is however limited often resulting in reduced dissolution rate of the active.

EP 1184038 discloses a system whereby a substance which is orally taken and to be delivered into the lower digestive tract is selectively delivered into the lower digestive tract. More particularly, a system which makes it possible to surely and quickly deliver the aimed substance to the lower digestive tract without being affected by pH change in the digestive tract due to change in bacterial flora is provided. This document discloses compositions that disintegrate in the lower digestive tract characterized by containing a compound <A>, which has a molecular weight of 1000 or less and has a disulfide bond, and a polymer, which has a molecular weight exceeding 1000 and is digested by enteric bacteria and/or undergoes softening, swelling or dissolution due to a decrease in pH. Also contemplated in this document are molded products with the use of these compositions and preparations with the use of these molded products.

There is a need therefore for an oral formulation or process which can be used to administer the solubilised and/or dispersions including nanosuspensions of the active ingredients in a manner which allows the formulation to be subsequently coated to deliver the active ingredient at a predetermined site of absorption and /or at a predetermined rate of delivery consistent with the optimum absorption and bioavailability or plasma profile of the drug.

Additionally there is a need for an oral formulation which can be used to administer one or more active ingredients of differing solubility which are released in a predetermined manner to target sites in the gastrointestinal tract so as to achieve maximum absorption at the site of release of active ingredient.

There is also a need for an oral formulation which allows for release of an active ingredient in the gastrointestinal tract in a manner which minimises high local concentrations of solid active ingredient. Multiparticulate drug delivery systems by their nature allow the release of the active ingredient over a larger surface area of the gastrointestinal tract thereby minimising high localised drug concentration for drugs which are irritants to the gastrointestinal tract.

Drugs which are poorly permeable are often administered with one or more permeability enhancers to enhance their permeability and absorption. Numerous potential absorption enhancers have been identified. Medium chain fatty acids and triglycerides have demonstrated the ability to enhance the absorption of hydrophilic drugs across the gastrointestinal mucosa (Pharm.Res., 1994,11,1148-54), (Pharm. Res.1993, 10,857-864). U.S. Patent No. 4,656,161 (BASF AG) discloses a process for increasing the bioavailability of heparins and heparinoids by adding non-ionic surfactants with a fatty acid, a fatty alcohol, an alkylphenol or a sorbitan or glycerol fatty acid ester. It is also known that the administration of medium chain fatty acids and derivatives of, including amino acid derivatives in combination with the drug can enhance permeability of the drug.

For maximum enhancement, it is desirable that both enhancer and drug are in solution at the same rate. The solubilities of the enhancer and drug are often different resulting in different rates of solubilisation of the enhancer and drug and hence loss of bioavailability of the drug in comparison with bioavailability from a solution of the drug and enhancer. In addition poorly permeable drugs including macromolecular drugs and biotechnology drugs such as peptides, proteins, vaccines, oligosaccharides, polysaccharides including TRH, Calcitonin, Leoprolide acetate, alendronate, vasopressin, desmopressin and antisense oligonucleotides are acid labile and cannot be delivered to the stomach as a solution.

The use of essential oils to enhance bioavailability by reducing cytochrome P450 metabolism and/or P-glycoprotein regulated transport of the active out of the blood stream back into the gut is also known, (US 5,66,386 to AvMax Inc. and others).

There is a need therefore for a controlled release technology which will allow the delivery of a drug and enhancer in solution to the optimum site of absorption/action in the gastrointestinal tract.

### Summary Of The Invention

The invention provides a controlled release formulation in solid unit dosage form, said formulation comprising a multiplicity of seamless microcapsules, each of which microcapsule contains one or more active ingredient in a liquid phase. The microcapsules are obtainable by a method wherein two different solutions, which are not dissolved with each other or are hardly dissolved with each other, contact each other, the solutions being a core solution which is a hydrophobic solution or suspension of an active ingredient and a shell solution, and wherein a shell/core mixed suspension is processed through a nozzle having a single orifice, to form a capsule or a bead of shell/core mixed suspension which is then ejected into a cooling or hardening solution and wherein the shell solution is gelled or solidified, the core containing a surfactant as an auxiliary agent. Each microcapsule has a predetermined release rate of each active ingredient in the gastrointestinal tract following administration. The microcapsules collectively have one or more rates of release of each active ingredient dependent on a predetermined permeability of the respective microcapsules.

The invention also provides a process of seamless microcapsule formation wherein two different solutions, which are not dissolved with each other or are hardly dissolved with each other, contact each other, the solutions being a core solution which is a hydrophobic solution or suspension of an active ingredient and a shell solution, and wherein a shell/core mixed suspension is processed through a nozzle having a single orifice, to form a capsule or a bead of shell/core mixed suspension which is then ejected into a cooling or hardening solution and wherein the shell solution is gelled or solidified, the core containing a surfactant as an auxiliary agent.

The microcapsules can be administered as a sprinkle on food or filled into a hard gelatin capsule or tabletted as a matrix, bilayer or multiple layer tablets. The final dosage form can be further coated to release the microcapsules at predetermined rates and/or site of the gastrointestinal tract.

The microcapsules of the formulation according to the invention effectively allow one to administer solutions or suspensions of active ingredients as if they were multiparticulate solid oral dosage forms. The microcapsules release their contents to the gastrointestinal tract in a manner which minimises high local concentrations of active ingredient which might otherwise result in irritation and other undesirable side effects, but additionally the drug is released in an already solubilised form which aids absorption.

The individual microcapsules suitably have an average diameter in the range 100-10,000µm, more particularly in the range 250-8,000µm and especially in the range 500-5,000µm_{.}

The walls of the microcapsules have a predetermined permeability by which is meant they either dissolve or have a natural permeability to gastrointestinal fluid so that active ingredient is released therefrom as desired in the gastrointestinal tract.

Microcapsules with naturally permeable walls can be soluble, porous or, alternatively, the solubility or porosity can develop as a result of the change in environmental conditions as the formulation passes through the gastrointestinal tract.

Thus, the wall of each microcapsule can be formed of a pharmaceutically acceptable, film forming polymer or mixture of polymers which is soluble in the gastrointestinal tract.

In a preferred embodiment, the wall of the microcapsule is formed of soft or hard gelatin (such as a bovine or porcine gelatin material) or other soft gel materials made from suitable polymers. Examples of other soft gel materials include starches that form a soft gel or high molecular weight polyethylene glycols or Agar/Agar. However, in practice any material that can dissolve in the gastrointestinal fluid can be used. Such a material can be a material which is incorporated in the wall and which dissolves in the gastrointestinal fluid, namely a porosigen.

The wall of the microcapsule will have a polymeric composition and/or structure which allows for fast release and thus fast absorption of active ingredient once the wall is partially or wholly permeable.

Once the wall of the microcapsule has the requisite permeability, the active ingredient in its liquid carrier passes into the gastrointestinal tract for immediate absorption. The liquid carrier can include one or more auxiliary agents with bioavailability and/or cytoprotective, especially gastroprotective, enhancing properties.

The formulation according to the invention can comprise a blend of microcapsules having walls of variable, but predetermined permeability so as to achieve immediate, intermediate or sustained release of active ingredient over a given time period in the gastrointestinal tract.

Accordingly, it will be appreciated that the formulation according to the invention has advantages over conventional solid microparticles in which the release of active ingredient is dependent on progressive solubilisation in the gastrointestinal tract.

The permeability of the walls of the microcapsules can be dependent on pH, temperature and other physical conditions prevailing within the gastrointestinal tract.

The controlled release properties of the microcapsules according to the invention will principally be a function of the thickness of the walls of the microcapsules, or by including pH dependent substances such as polymers or shellac.

The microcapsules can contain two or more active ingredients having different solubilities in the aqueous environment of the gastrointestinal tract, but with compatible solubility or suspending capability in the liquid medium of the microcapsule. Alternatively, the microcapsules can contain single active ingredients solubilised in different media but which can be released for simultaneous absorption from microcapsules having walls of different materials but with similar or different permeability characteristics.

The microcapsules can also contain two or more active ingredients having different half-lives following absorption from the gastrointestinal tract.

The solubility of the or each active ingredient can be dependent on the pH of a given region of the gastrointestinal tract.

The microcapsules can be manufactured so that they release their contents in the gastrointestinal tract at a point at which the drug is most soluble. This feature enables one to maximise absorption because the microcapsules release their contents when the pH conditions are optimal.

The pH internally of the microcapsules can be optimised by the use of an acid or an alkaline solution, as required to maximise the absorption of active ingredients that are pH sensitive.

An example of a formulation with microcapsules capable of achieving immediate, intermediate and sustained release of active ingredient over a given time period in the gastrointestinal tract might be a formulation for use in the treatment of the common cold and influenza. Such formulations are conventionally multiple active ingredient formulations. The common cold and influenza are each characterised by a variety of symptoms which cannot generally be alleviated by a single active ingredient. For example, it might be desired to administer an antihistamine, a decongestant and one or more cough suppressants.

It may also be desirable to add a diuretic such as hydrochlorothiazide and/or an antihypertensive such as Losartan.

The formulation according to the present invention is ideally suited for use as a multiple active ingredient formulation.

The microcapsules can contain an active ingredient suspended or solubilised in a solution of a permeability enhancer in a ratio of enhancer to drug ranging from 0 drug: 100 enhancer to 100 drug : 0 enhancer which can be released for simultaneous absorption from microcapsules having walls of different materials but with similar or different permeability characteristics.

Additionally microcapsules containing the enhancer solution without the drug can be released at a slower rate from microcapsules containing the drug and enhancer solutions to maximise the permeability enhancement of the unabsorbed drug in the gastrointestinal tract.

The walls of the microcapsules can have inherent mucoadhesive properties and thus bind to the wall of the gastrointestinal tract during release of active ingredient therefrom.

The walls of the microcapsules can also have inherent enteric coating properties.

Some or all of the microcapsules in the formulation can have an enteric coating, for example, an outer membrane of shellac or other enteric coating.

It will be appreciated that one can achieve selective absorption of active ingredients using a formulation in accordance with the invention.

Essentially the solution in which the drug is dissolved or suspended can be any pharmaceutically acceptable solvent or liquid phase provided the solvent or liquid phase does not dissolve the wall of microcapsules.

The liquid phase can suitably be an oil. When the oil is soya bean oil or mineral oil, the active agent would typically form a suspension in such oils. The oil phase can be an oil of nutritional benefit or having pharmacological activity such as omega-3-fatty acids and omega-6 fatty acids

Such liquid phases include, for example, short or medium chain mono-, di and/or triglycerides.

Suitably the or each active ingredient can be dissolved in a polyol in the core of the microcapsules. Examples of polyols are polyethylene glycols and cellulose derivatives.

The core can also contain one or more auxiliary agents selected from a pH controlling agent, an anti-oxidant, a humectant, a surfactant and a vasodilator.

Suitable pH controlling agents include, for example, citric acid, fumaric acid, sodium citrate and the like.

Examples of anti-oxidants are sodium metabisulphite, butylated hydroxyanisole and butylated hydroxytoluene or a mixture thereof.

Examples of suitable humectants are glycerol and sorbitol.

Examples of suitable surfactants include sodium lauryl sulphate, diethylene glycol monostearate, propylene glycol monosteareate, polyethylene glycol, polysorbates and polyvinyl alcohol or mixtures thereof.

The microcapsules can contain up to 90% by weight of active ingredient. However, in general the microcapsules will contain between 25 and 75% by weight of active ingredient.

Each formulation will suitably contain between 10 and 300 microcapsules, preferably between 100 and 250 microcapsules.

The microcapsules will suitably be administered by loading them into a hard gelatin capsule which will be swallowed in the normal way or by loading them into another container such as a sachet, the contents of which can then be swallowed or sprinkled onto food and swallowed. In certain special circumstances the microcapsules may be incorporated into a tablet in a way which maintains their integrity.

A formulation in accordance with the invention can be designed so as to achieve fast, medium and slow release of one or more active ingredients. Thus, it will be appreciated that the formulation in accordance with the invention can be used to achieve maximum bioavailability resulting from maximum absorption of one or more active ingredients from the gastrointestinal tract.

The formulation according to the invention is suitable for the administration of a wide range of active ingredients.

For example the formulation can be used in the case of insoluble active ingredients such as nifedipine, lipid soluble active ingredients such as gemfibrizol, and pH sensitive active ingredients such as captopril.

The formulation according to the invention is also suitable for the administration of active ingredients which are sensitive to the pH environment in the stomach, such as omeprazole and other proton pump inhibitors used in anti -ulcer treatment

The formulation according to the invention can also be used to improve the bioavailability of active ingredients such as terfenadine which have a low oral bioavailability.

The formulation according to the invention can also be used to dramatically increase the absorption of active ingredients which are poorly absorbed from or are destroyed in the gastrointestinal tract such as captopril, cyclosporin, calcitonin, heparins and heparinoids.

Suitable classes of therapeutic agents which can be delivered using this invention include but are not limited to poorly water soluble drugs such as cardiovascular agents, lipid lowering agents, anti-diabetic agents, anti-epileptics, anti-infectives, anti-fungal agents, anti-viral agents, antipsychotic agents, immunosuppressants, protease inhibitors, cyclic peptides.

Suitable classes of therapeutic agents which can be delivered using this invention include but are not limited to peptides, proteins, vaccines, and oligonucleotides, including non-covalent or covalent modified versions thereof.

An example of a formulation of an insoluble drug providing both immediate and sustained release in accordance with the invention comprises a capsule containing a mixture of immediate and sustained release seamless microcapsules of varying sizes, for example. 1-8mm. The active ingredient, for example, nifedipine or cyclosporine is dissolved or suspended, as appropriate, for example, in a polyethylene glycol, an oil-base, a suspension of a polyethylene glycol and a mineral oil, or polyoxethylene sorbitan fatty acid esters or a suitable combination of an oil and surfactants or an emulsion or microemulsion preconcentrate. An outer membrane liquid for example, gelatin and an inner active ingredient liquid are combined to form droplets i.e., microcapsules. The microcapsules are passed through a cooling system, for example, oil. The seamless microcapsules containing the active ingredient are removed from the cooling system and cleaned and dried in separate facilities.

The sustained action of the microcapsules is determined by the thickness of the outer membrane or by the addition of a variety of sustained release polymers to the inner liquid or the outer membrane liquid, for example, polymethacrylates, cellulose derivatives, polyvinylpyrrolidones. As indicated above, the microcapsules can be formulated to release the active liquid ingredient at a specific absorption site, for example by the use of pH dependent polymethacrylates.

The invention provides an oral formulation which allows for protection of the active ingredient from harsh environments such as gastric acid and intestinal proteases and other degradative processes. Enteric coating protects drugs from release into acidic environments, protease and nuclease inhibitors reduce proteolytic and nucleic acid degradation while mucoadhesive coatings minimise exposure to degradative enzymes.

For maximum protection from degradative processes, it may be desirable that the drug to be protected is formulated as a solution or emulsion that contains the appropriate degradation inhibitor, such as a nuclease or protease inhibitor. Otherwise, it may be necessary to formulate the drug and degradative process inhibitor as separate minicapsules. In addition, it may be necessary to coat the minicapsules with both an outer enteric coat and an inner mucoadhesive coating to reduce the exposure time to degradative enzymes thereby mitigating degradation.

The invention will be further illustrated by the following examples:

### General Outline of Seamless Microcapsule Formation

The principle of seamless microcapsule formation is the utilisation of "surface tension," when two different solutions (which are not or hardly dissolved with each other) contact each other, which works by reducing the contact area of the two different solutions.

After encapsulating the core solution, which is ejected through an orifice with a certain diameter, with the shell solution which is also ejected through an outer orifice, the encapsulated sphere is then ejected into a cooling or hardening solution and the outer shell solution is gelled or solidified. Thus seamless microcapsules are formed.

The core solution is mainly a hydrophobic solution or suspension. The outer shell solution is normally gelatin based. However, a hydrophilic solution can also be encapsulated with the existence of an intermediate solution, which can avoid the direct contact of the hydrophilic core solution with the outer shell.

With the nozzle having a single orifice, a capsule or a bead of shell/core mixed suspension can be processed. With the nozzle having two orifices (center and outer), a hydrophobic solution can be encapsulated. With the nozzle having three or more orifices seamless microcapsules for various applications can be processed.

The following Examples describe the manufacture of seamless microcapsules according to the above described principle of formation. It will be recalled, however, that the claims of this specification are confined to processes where a shell/core mixed suspension is processed through a nozzle having a single orifice and to controlled release formulations comprising microcapsules obtainable by such a process.

### Example 1

Nifedipine is dissolved in soybean oil and formed into seamless microcapsules according to the methods described in US Pat. Nos. 5,478,508 and 5,882,680 with an outer gelatin coating as hereinbefore described. These microcapsules have a size distribution of 1-3mm.

The nifedipine microcapsules are then coated in a conventional manner with a cellulose polymer coating to provide a controlled release dissolution rate.

The coated microcapsules are finally encapsulated into a hard gelatin capsule shell.

The details for this example are as follows:

| *Core Solution* | *Weight %* |
|---|---|
| -Nifedipine USP | 9.1 |
| -Polyethylene Glycol (Grade 200;300;400;600) | 90.9 |
| *Intermediate Solution* | |
| Vegetable Oil | 100.0 |
| *Film Solution* | |
| -- Gelatin | 12.0 - 22.5 |
| -- Sorbitol | 1.5 - 2.5 |
| Purified Water | 75 - 85 |

### Microcapsule Formation

| *Ingredients* | *Weight %* |
|---|---|
| -- Core Solution | 30-70 |
| Intermediate Solution | 5-25 |
| Film Solution | 15-65 |

The core solution is formed into seamless microcapsules with an intermediate solution layer and an outer hard gelatin film layer. The intermediate layer acts as a barrier preventing the core solution from migrating into the outer gelatin layer.

The microcapsules produced have a particle size range of between 1.00 - 3.00 mm.

Nifedipine Mean Dissolution Rate (1.25% sodiumlaurylsulphate)

| *Time* | *Mg*/*Release* | *% Release* |
|---|---|---|
| 5 min | 93.2 | 28.6 |
| 10min | 190.3 | 58.4 |
| 15 min | 230.5 | 70.8 |
| 30 min | 279.1 | 85.7 |
| 45 min | 324.8 | 99.8 |

### Example 2

Nifedipine seamless microcapsules are formulated as described in Example 1, however, the inherent release characteristics of the pellets are varied from Example 1.

These microcapsules provide a longer controlled release action.

The details for this example are as follows:

### Ingredients

- Nifedipine Microcapsules (1.50 - 1.80 mm) 500 Grams
- Ammino Methacrylate Copolomer (Eudragit® RL) 5-50 w/w
- Ammino Methacrylate Copolomer (Eudragit® RS) 50-95 w/w
- Isopropyl Alcohol* ---
- Acetone* ---
*Used in processing, occurring in trace amounts in finished product

Place 500 grams of Nifedipine microcapsules prepared in the same way as Example 1 in a suitable fluidised bed system (eg Glatt GPCG 1 or Vector FL-M-1 Unit).Spray coat the microcapsules with a 6.25 solution comprising Eudragit® RL (10% w/w) and Eudragit® RS (90% w/w) dissolved in isopropyl alcohol/acetone. Talc was added simultaneously via an auger feeder to prevent agglomeration.

The resulting product consisted of a microcapsule coated with a rate -controlling polymer that provides a sustained release of Nifedipine throughout the gastrointestinal tract over a 12 or 24 hour duration. The coated microcapsules are then filled into hard gelatin capsules.

### Example 2A

Nifedipine seamless microcapsules were prepared in the same way as for the microcapsules in Example 1, however the inherent release characteristics of the microcapsules are varied from Example 1, by increasing the wall thickness of the outer gelatin layer of the microcapsule.

The microcapsules were placed in a suitable fluidised bed coater and spray coated with the same polymer coating formulation as in Example 2, thus providing a longer sustained release action typically 24 hours.

### Example 3

Gemfibrozil (a liquid soluble drug) is formulated along with various surfactants and gelatin into seamless microcapsules of varying thickness.

A portion of these pellets are coated with a methacrylate polymer system and combined in a ratio of 4:1 with uncoated microcapsules, then filled into hard gelatin capsule shells, thereby providing a drug formulation having both immediate and sustained release dissolution characteristics.

### Example 4

Captopril is dissolved in soy bean oil, and formed into seamless microcapsules with an outer gelatin coating. These microcapsules have a size distribution of 1-3mm.

The microcapsules are then coated in a conventional manner with a cellulose polymer coating to provide a controlled release dissolution rate.

The coated microcapsules are finally encapsulated into a hard gelatin capsule shell.

### Example 4A

### Core Solution

- Captopril 60 grams
- Polyethylene Glycol (grade 200;300;400;600) 100 grams
- Citric Acid Anhydrous pH Adjuster
- Purified Water 100 grams

### Intermediate Solution

As in Example 1

### Film Solution

As in Example 1

Place 100 grams of Polyethylene Glycol (grade 200;300;400;600) and 100 grams Purified Water in a suitable container and mix using a mechanical mixer. Add a suitable quantity of Citric Acid to the Glycol/Water mix, to bring to predetermined pH value. Add the Captopril to the solution. Additional Citric Acid maybe added if the predetermined pH value is not achieved.

The Theoretical Target Potency of the Core Solution should be in the range of 200 - 300 mg/g.

The seamless microcapsules are produced as described in Examples 1 and 2.

### Example 5

Captopril seamless microcapsules are formulated as described in Example 4, however, the inherent release characteristics of the microcapsules is varied from Example 4, by increasing the wall thickness of the microcapsules.

These microcapsules, when coated with the same polymer coating as in Example 4, provide a longer controlled release action.

### Example 6

Cyclosporine is solubilised in a suitable medium chain triglyceride and formed into seamless microcapsules with an outer gelatin coating as herein before described. These microcapsules have a size distribution of 3-6mm.

The cyclosporine microcapsules are then encapsulated into a hard gelatin capsule shell.

### Example 7

Cyclosporine dispersion is prepared and formed into seamless microcapsules with an outer gelatin coating as herein before described. These microcapsules have a size distribution of 3-6mm.

The cyclosporine microcapsules are then encapsulated into a hard gelatin capsule shell.

### Example 8

Cyclosporine is solubilised in a suitable medium chain triglyceride, and formed into seamless microcapsules with an outer gelatin coating as herein before described. These microcapsules have a size distribution of 3-6mm.

Omega-3 fatty acids are encapsulated into seamless microcapsules as described in Example 1. The cyclosporine microcapsules and omega-3 fatty acid microcapsules are mixed in the ratio of 100:300 and filled into hard gelatin capsules to give a cyclosporine content of 25mg /capsule

The cyclosporine microcapsules are then encapsulated into a hard gelatin capsule shell.

### Example 8A

Cyclosporine is solubilised in a suitable medium chain triglyceride and formed into seamless microcapsules with an outer gelatin layer as described in Example 1 and in the basic technology description alone. The microcapsules produced have a particle size distribution in the range 1.00-3.00mm.

Omega-3-fatty acids are produced into seamless microcapsules as described in Example 1 and the basic technology description. The microcapsules produced have a particle distribution in the range 1.00-3.00mm.

The cyclosporine microcapsules and omega-3-fatty acid microcapsules are encapsulated into hard gelatin capsules using a Macofar Multi-Dosing Pellet Encapsulating Machine in the ratio 100:300 to give a Cyclosporine Active Content of 25mg/capsule.

### Example 9

Nimodipine is solubilised in a suitable solvent such as PEG 400 and formed into seamless microcapsules with an outer gelatin coating as hereinbefore described. These microcapsules have a size distribution of 3-6mm.

The nimodipine microcapsules are then encapsulated into a hard gelatin capsule shell.

### Example 10

Nimodipine seamless microcapsules are formulated as described in Example 9, however, the inherent release characteristics of the pellets are varied from Example 9, by increasing the wall thickness of the microcapsules.

These microcapsules, when coated with the same polymer coating as in Example 1, provide a longer controlled release action.

### Example 11

Cyclosporine is solubilised in a suitable medium chain triglyceride to which is added bile salt enhancer or bile salts, and formed into seamless microcapsules with an outer gelatin coating as herein before described. These microcapsules have a size distribution of 3-6mm.

The cyclosporine and bile salt enhancer or bile salts microcapsules are then encapsulated into a hard gelatin capsule shell to give a cyclosporine content of 25-50mg /capsule.

### Example 12

Cyclosporine is solubilised in a suitable medium chain triglyceride, and formed into seamless microcapsules with an outer gelatin coating as herein before described. These microcapsules have a size distribution of 3-6mm.

Bile salt enhancer or bile salts are encapsulated into seamless microcapsules as described in Example 1. The cyclosporine microcapsules and bile salt enhancer or bile salt microcapsules are mixed and filled into hard gelatin capsules to give a cyclosporine content of 25-50mg /capsule

The cyclosporine and bile salt enhancer or bile salts microcapsules are then encapsulated into a hard gelatin capsule shell.

### Example 13

Separate cyclosporine and bile salt enhancer or bile salt solutions are prepared as per Example 12.

The respective cyclosporine and bile salt enhancer or bile salt microcapsules are coated as in Example 1 to provide a different controlled release profiles. The bile salt enhancer or bile salts released prior to release of the cyclosporine.

### Example 14

A P-glycoprotein inhibitor such as cyclosporine is solubilised in a suitable medium chain triglyceride, and formed into seamless microcapsules with an outer gelatin coating as herein before described. These microcapsules have a size distribution of 3-6mm.

P-glycoprotein sensitive drugs such as bioflavonoids or antineoplastic molecules such as paclitaxel are encapsulated into seamless microcapsules as described in Example 1. The cyclosporine microcapsules and P-glycoprotein sensitive drug microcapsules are mixed and filled into hard gelatin capsules.

Ref:-Inhibition of P-glycoprotein by flavonoid derivatives in adriamycin-resistant human myelogenour leukaemia (K562/ADM) cells, Cancer Lett.2002 Mar 8;177(1):89-93

### Example 15

A blood brain barrier permeability modulators such as cyclosporine is solubilised in a suitable medium chain triglyceride, and formed into seamless microcapsules with an outer gelatin coating as herein before described. These microcapsules have a size distribution of 3-6mm.

Drugs with limited blood brain barrier permeability such as Nimodipine are encapsulated into seamless microcapsules as described in Example 1.

The blood brain barrier permeability modulator microcapsules and limited blood brain barrier permeability compound microcapsules are mixed and filled into hard gelatin capsules.

*Ref:-* P-glycoprotein restricted transport of Nimodipine across blood brain barrier,Acta Pharmacol Sin. 2003 Sep,24(9);903-6

### Example 16

An intestinal permeability modulator such as nifedipine is formulated as in Example 1 and formed into seamless microcapsules with an outer gelatin coating as herein before described. These microcapsules have a size distribution of 3-6mm.

A drug with limited intestinal permeability such as cyclosporine is solubilised in a suitable medium chain triglyceride, and formed into seamless microcapsules with an outer gelatin coating as herein before described. These microcapsules have a size distribution of 3-6mm.

The intestinal permeability modulator microcapsules and compound with limited intestinal permeability microcapsules are mixed and filled into hard gelatin capsules.

*Ref:-* Nifedipine improves immediate, 6 and 12 month graft function in cyclosporine (CyA) treated renal allograft recipients, TransplInt. 1992;5 Suppl 1;S69-72

### Example 17

A P-glycoprotein inhibitor such as cyclosporine is solubilised in a suitable medium chain triglyceride, blended with a suitable pH modulator such as sodium citrate, and formed into seamless microcapsules with an outer gelatin coating as herein before described. These microcapsules have a size distribution of 3-6mm.

A P-glycoprotein and pH-sensitive drug such as Berberine is encapsulated into seamless microcapsules as described in Example 1. The cyclosporine microcapsules and P-glycoprotein/pH-sensitive drug microcapsules are mixed and filled into hard gelatin capsules.

*Ref:-*Transport and uptake characteristics of a new derivative of berberine (CPU-86017 in human intestinal epithelial cell line: Caco-2, Acta Pharmacol Sin.2003 Dec, 24(12):1185-91

### Example 18

A hydroxymethyl-glutaryl-coenzyme (HMG-CoA) reductase inhibitor such as simvastatin is encapsulated into seamless microcapsules as described in Example 1.

A RES stimulator such as complex carbohydrate is solubilised and formed into seamless microcapsules with an outer gelatin coating as herein before described. These microcapsules have a size distribution of 3-6mm.

The hydroxymethyl-glutaryl-coenzyme (HMG-CoA) reductase inhibitor microcapsules and RES stimulator microcapsules are mixed and filled into hard gelatin capsules.

### Example 19

| | |
|---|---|
| Cyclosporine USP | 100 |
| Labrafilt M 1944 | 50-300 |
| Cremophor® RH40 | 50-400 |
| Tween® 80 | 0-200 |
| Polyethylene Glycol (grade 200;300;400;600) | 50-200 |
| Ethanol | --- |

Place Labrafil®, Cremophor® and Tween® (if required) in a suitable container and mix using a mechanical mixer until uniformly dispersed or dissolved. (Label as Mix 1)

Place polyethylene glycol and ethanol (as required) in a suitable container. Add cyclosporine and mix using a mechanical mixer until the cyclosporine is completely solubilised. (Label as Mix 2)

Add Mix 1 to Mix 2 and continue mixing until all ingredients are uniformly dispersed. This is the Active Core Solution.

The active core solution is processed in the same way as described in Example 1 to form seamless microcapsules having a particle size range of 1.50-2.00mm.

The cyclosporine microcapsules are then encapsulated into hard gelatin capsules.

### Example 20

The microcapsules of Example 19 were administered to 8 healthy male volunteers and the bioavailability was compared with a conventional soft gel formulation of cyclosporin which is available under the Trade Mark Sandimmune® from Novartis.

The resulting bioavailability data demonstrate that the Tmax was reduced from 3.25 hr to 1.75 hr (100% increased speed to uptake) and the Cmax was increased from 580ng/ml to 750ng/ml (>40% increased drug uptake) when compared with the conventional formulation. Overall the AUC (area under the curve) was increased more than 20% when compared with the reference formulation. The data is presented in the following table. The bioavailability data are illustrated in Fig. 1 in which the continuous line represents bioavailability of the product of Example 19 and the interrupted line is bioavailability of the conventional reference product.

| PK Parameters | | **Formulation of example 19** | **Reference** | **Summary** |
|---|---|---|---|---|
| **Relative** | **Based** | **103.14 ±44.62** | | **-** |
| **Bioavailability (%)** | **on AUCinf** | **43.3** | | **-** |
| **AUCinf (ng/mL.h) CV%** | | **3040.00 ± 1091.71** | **2586.23 ± 341.15** | **> 20%** |
| | | **35.9** | **13.2** | **Increase** |
| **AUClast (ng/mL.h)** | | **2855.09 ± 1043.08 36.5** | **2410.68 ± 332.93** | **> 20% Increase** |
| **CV%** | | | **13.8** | |
| **Cmax (ng/mL) CV%** | | **732.31 ± 273.67** | **581.06 ± 109.13** | **-40% Increase** |
| | | **37.4** | **18.8** | |
| **Tmax(h) CV%** | | **2.00 ± 0.93** | **3.00 ± 1.31** | **100%** |
| | | **46.3** | **43.6** | **Faster** |
| **Median Range** | | **1.75 (1.00-3.50)** | **3.25 (1.00-5.00)** | |
| **Thalf (h) CV%** | | **8.12 ± 0.50** | **7.95 ± 1.30** | **Equivalent** |
| | | **6.1** | **16.3** | |

The present invention allows for an active pharmaceutical ingredient to be maintained in a liquid phase and then encapsulated into microcapsules. The present invention also allows the active ingredient to be maintained in its liquid phase thus enhancing the solubility of an insoluble partially soluble pharmaceutical ingredient.

## Claims

1. A process of seamless microcapsule formation wherein two different solutions, which are not dissolved with each other or are hardly dissolved with each other, contact each other, the solutions being a core solution which is a hydrophobic solution or suspension of an active ingredient and a shell solution, and wherein a shell/core mixed suspension is processed through a nozzle having a single orifice, to form a capsule or a bead of shell/core mixed suspension which is then ejected into a cooling or hardening solution and wherein the shell solution is gelled or solidified, the core containing a surfactant as an auxiliary agent.

2. A process of claim 1 wherein the shell solution is gelatin based.

3. A process of claim 1 or claim 2 wherein the microcapsule has a diameter of from 0.5 mm to 5 mm.

4. A process of any preceding claim which further comprises coating the microcapsule with a material selected from a cellulose polymer coating and an enteric coating

5. A process of any preceding claim wherein the shell solution comprises a sustained release polymer.

6. A process of any preceding claim which further comprises forming a multiplicity of the seamless microcapsules into a controlled release formulation in solid unit dosage form, optionally wherein the solid unit dosage form is a hard gelatine capsule.

7. A controlled release formulation in solid unit dosage form, said formulation comprising a multiplicity of seamless microcapsules, each microcapsule comprising one or more active ingredients in a liquid phase, and being obtainable by a method wherein two different solutions, which are not dissolved with each other or are hardly dissolved with each other, contact each other, the solutions being a core solution, which is a hydrophobic solution or suspension of an active ingredient and a shell solution, and wherein a shell/core mixed suspension is processed through a nozzle having a single orifice, to form a capsule or a bead of shell/core mixed suspension which is then ejected into a cooling or hardening solution and wherein the shell solution is gelled or solidified, the core containing a surfactant as an auxiliary agent;
and each microcapsule having a predetermined release rate of each said active ingredient in the gastrointestinal tract following administration, the microcapsules collectively having one or more rates of release of each said active ingredient dependent on a predetermined permeability of the respective microcapsules.

8. A formulation of claim 7 wherein the shell solution is gelatin based.

9. A formulation of claim 7 or claim 8 wherein one or more active ingredients are dissolved in the pharmaceutically acceptable liquid phase.

10. A formulation of any of claims 7 to 9 wherein the shell solution additionally includes a sustained release polymer and/or wherein some or all of the microcapsules in the formulation have an enteric coating.

11. A formulation of any of claims 7 to 10 wherein the microcapsules have an average diameter in the range of 0.5-5mm.

12. A formulation of claim 9 wherein the microcapsules further comprise a cellulose polymer coating.

13. A formulation of any of claims 7 to 12 further comprising a feature selected from (a), (b) and (c):
(a) the formulation is a multiple active ingredient formulation;
(b) the microcapsules contain two or more active ingredients having different solubilities in the aqueous environment of the gastrointestinal tract;
(c) the microcapsules contain two or more active ingredients having different half lives following absorption from the gastrointestinal tract.

14. The subject matter of any preceding claim wherein the surfactant is selected from sodium lauryl sulphate, diethylene glycol monostearate, propylene glycol monosteareate, polyethylene glycol, polysorbates and polyvinyl alcohol or a mixture thereof.

15. The subject matter of any preceding claim wherein the active ingredient is a drug and optionally is selected from nifedipine, gemfibrizol, terfenadine, captopril, cyclosporin, calcitonin, heparins and heparinoids, e.g. wherein the active ingredient is cyclosporin, or wherein the active ingredient is a therapeutic agent selected from peptides, proteins, vaccines, and oligonucleotides.

16. The subject matter of any of claims 1 to 14, wherein the active ingredient is nimodipine.

## Patentansprüche

1. Verfahren zur Bildung nahtloser Mikrokapseln, wobei zwei unterschiedliche Lösungen, die nicht miteinander gelöst sind oder kaum miteinander gelöst sind, einander kontaktieren, wobei die Lösungen eine Kernlösung, die eine hydrophobe Lösung oder Suspension eines Wirkstoffs ist, und eine Hüllenlösung sind, und wobei eine Hüllen-/Kern-Mischsuspension durch eine Düse verarbeitet wird, die eine einzelne Öffnung aufweist, um eine Kapsel oder eine Kugel der Hüllen-/Kern-Mischsuspension zu bilden, die dann in eine Kühl- oder Härtelösung ausgestoßen wird und wobei die Hüllenlösung geliert oder verfestigt wird, wobei der Kern ein Tensid als Hilfsstoff enthält.

2. Verfahren gemäß Anspruch 1, wobei die Hüllenlösung Gelatine basiert ist.

3. Verfahren gemäß Anspruch 1 oder Anspruch 2, wobei die Mikrokapsel einen Durchmesser von 0,5 mm bis 5 mm aufweist.

4. Verfahren gemäß einem vorhergehenden Anspruch, das weiterhin das Beschichten der Mikrokapsel mit einem Material umfasst, das aus einer Cellulose-Polymer-Beschichtung und einer enterischen Beschichtung ausgewählt ist.

5. Verfahren gemäß einem vorhergehenden Anspruch, wobei die Hüllenlösung ein Polymer mit verzögerter Freisetzung umfasst.

6. Verfahren gemäß einem vorhergehenden Anspruch, das weiterhin das Bilden einer Vielzahl der nahtlosen Mikrokapseln in einer Formulierung mit kontrollierter Freisetzung in fester Einheitsdosierungsform umfasst, wobei optional die feste Einheitsdosierungsform eine harte Gelatinekapsel ist.

7. Formulierung mit kontrollierter Freisetzung in fester Einheitsdosierungsform, wobei die Formulierung eine Vielzahl von nahtlosen Mikrokapseln umfasst, wobei jede Mikrokapsel einen oder mehrere Wirkstoffe in einer Flüssigphase umfasst, und durch ein Verfahren gewonnen werden kann, bei dem zwei unterschiedliche Lösungen, die nicht miteinander gelöst sind oder kaum miteinander gelöst sind, einander kontaktieren, wobei die Lösungen eine Kernlösung, die eine hydrophobe Lösung oder Suspension eines Wirkstoffs ist, und eine Hüllenlösung sind, und wobei eine Hüllen-/Kern-Mischsuspension durch eine Düse verarbeitet wird, die eine einzelne Öffnung aufweist, um eine Kapsel oder eine Kugel der Hüllen-/Kern-Mischsuspension zu bilden, die dann in eine Kühl- oder Härtelösung ausgestoßen wird und wobei die Hüllenlösung geliert oder verfestigt wird, wobei der Kern ein Tensid als Hilfsstoff enthält;
und wobei jede Mikrokapsel eine vorbestimmte Freisetzungsrate von jedem Wirkstoff im Gastrointestinaltrakt nach der Verabreichung aufweist, wobei die Mikrokapseln kollektiv eine oder mehrere Freisetzungsraten von jedem Wirkstoff aufweisen, abhängig von einer vorbestimmten Permeabilität der jeweiligen Mikrokapseln.

8. Formulierung gemäß Anspruch 7, wobei die Hüllenlösung Gelatine basiert ist.

9. Formulierung gemäß Anspruch 7 oder Anspruch 8, wobei ein oder mehrere Wirkstoffe in der pharmazeutisch akzeptablen Flüssigphase gelöst sind.

10. Formulierung gemäß einem der Ansprüche 7 bis 9, wobei die Hüllenlösung zusätzlich ein Polymer mit verzögerter Freisetzung einschließt und/oder wobei einige oder alle Mikrokapseln in der Formulierung eine enterische Beschichtung aufweisen.

11. Formulierung gemäß einem der Ansprüche 7 bis 10, wobei die Mikrokapseln einen durchschnittlichen Durchmesser im Bereich von 0,5-5 mm aufweisen.

12. Formulierung gemäß Anspruch 9, wobei die Mikrokapseln weiterhin eine Cellulose-Polymer-Beschichtung umfassen.

13. Formulierung gemäß einem der Ansprüche 7 bis 12, die weiterhin ein Merkmal umfasst, das aus (a), (b) und (c) ausgewählt ist:
(a) die Formulierung eine Mehrfach-Wirkstoff-Formulierung ist;
(b) die Mikrokapseln zwei oder mehrere Wirkstoffe enthalten, die unterschiedliche Löslichkeiten in der wässrigen Umgebung des Gastrointestinaltrakt aufweisen;
(c) die Mikrokapseln zwei oder mehrere Wirkstoffe enthalten, die unterschiedliche Halbwertszeiten nach Absorption vom Gastrointestinaltrakt aufweisen.

14. Gegenstand gemäß einem vorhergehenden Anspruch, wobei das Tensid aus Natriumlaurylsulphat, Diethylenglycolmonostearat, Propylenglycolmonosteareat, Polyethylenglycol, Polysorbaten und Polyvinylalkohol oder einer Mischung davon ausgewählt ist.

15. Gegenstand gemäß einem vorhergehenden Anspruch, wobei der Wirkstoff ein Arzneimittel ist und optional aus Nifedipin, Gemfibrizol, Terfenadin, Captopril, Cyclosporin, Calcitonin, Heparinen und Heparinoiden ausgewählt ist, z. B. wobei der Wirkstoff Cyclosporin ist, oder wobei der Wirkstoff ein therapeutisches Mittel ist, das aus Peptiden, Proteinen, Vakzinen und Oligonukleotiden ausgewählt ist.

16. Gegenstand gemäß einem der Ansprüche 1 bis 14, wobei der Wirkstoff Nimodipin ist.

## Revendications

1. Procédé de formation de microcapsules sans soudure dans lequel deux solutions différentes, qui ne sont pas dissoutes l'une avec l'autre ou ne sont pratiquement pas dissoutes l'une avec l'autre, se touchent, les solutions étant une solution de coeur qui est une solution hydrophobe ou une suspension d'un ingrédient actif et une solution de coque, et dans lequel une suspension mélangée de coque/coeur est traitée par le biais d'une buse possédant un seul orifice, pour former une capsule ou une bille de suspension mélangée de coque/coeur qui est ensuite éjectée en une solution de refroidissement ou de durcissement et dans lequel la solution de coque est gélifiée ou solidifiée, le coeur contenant un surfactant comme agent auxiliaire.

2. Procédé selon la revendication 1 dans lequel la solution de coque est à base de gélatine.

3. Procédé selon la revendication 1 ou 2 dans lequel la microcapsule a un diamètre de 0,5 mm à 5 mm.

4. Procédé selon l'une quelconque des revendications précédentes comprenant en outre l'enrobage de la microcapsule avec une matière choisie entre un enrobage polymère à base de cellulose et un enrobage entérique.

5. Procédé selon l'une quelconque des revendications précédentes dans lequel la solution de coque comprend un polymère à libération prolongée.

6. Procédé selon l'un quelconque des revendications précédentes comprenant en outre la formation d'une multiplicité de microcapsules sans soudure dans une formulation à libération contrôlée sous forme d'une dose unitaire solide, la dose unitaire solide étant éventuellement une capsule de gélatine dure.

7. Formulation à libération contrôlée sous forme de dose unitaire solide, ladite formulation comprenant une multiplicité de microcapsules sans soudure, chaque microcapsule comprenant un ou plusieurs ingrédients actifs en phase liquide, et pouvant être obtenue par un procédé dans lequel deux solutions différentes, qui ne sont pas dissoutes l'une avec l'autre ou ne sont pratiquement pas dissoutes l'une avec l'autre, se touchent, les solutions étant une solution de coeur qui est une solution hydrophobe ou une suspension d'un ingrédient actif et une solution de coque, et dans lequel une suspension mélangée de coque/coeur est traitée par le biais d'une buse possédant un seul orifice, pour former une capsule ou une bille de suspension mélangée de coque/coeur qui est ensuite éjectée en une solution de refroidissement ou de durcissement et dans lequel la solution de coque est gélifiée ou solidifiée, le coeur contenant un surfactant comme agent auxiliaire ;
et chaque microcapsule ayant un taux de libération prédéterminé de chacun desdits ingrédients actifs dans le tractus gastro-intestinal après administration, les microcapsules ayant collectivement un ou plusieurs taux de libération de chacun desdits ingrédients actifs en fonction d'une perméabilité prédéterminée des microcapsules respectives.

8. Formulation selon la revendication 7 dans laquelle la solution de coque est à base de gélatine.

9. Formulation selon la revendication 7 ou 8 dans laquelle un ou plusieurs ingrédients actifs sont dissouts dans la phase liquide pharmaceutiquement acceptable.

10. Formulation selon l'une quelconque des revendications 7 à 9 dans laquelle la solution de coque comprend en outre un polymère à libération prolongée et/ou dans laquelle certaines ou toutes les microcapsules dans la formulation ont un enrobage entérique.

11. Formulation selon l'une quelconque des revendications 7 à 10 dans laquelle les microcapsules ont un diamètre moyen de l'ordre de 0,5 à 5 mm.

12. Formulation selon la revendication 9 dans laquelle les microcapsules comprennent en outre un enrobage polymère à base de cellulose.

13. Formulation selon l'une quelconque des revendications 7 à 12 comprenant en outre une caractéristique sélectionnée parmi (a), (b) et (c) :
(a) la formulation est une formulation à multiples ingrédients actifs ;
(b) les microcapsules contiennent deux ingrédients actifs ou plus présentant des solubilités différentes dans l'environnement aqueux du tractus gastro-intestinal ;
(c) les microcapsules contiennent deux ingrédients actifs ou plus ayant des demi-vies différentes après absorption par le tractus gastro-intestinal.

14. Objet de l'une quelconque des revendications précédentes dans lequel le surfactant est choisi parmi le laurylsulfate de sodium, le monostéarate de diethylène glycol, le monostéarate de propylène glycol, le polyéthylène glycol, les polysorbates et les alcools polyvinyliques ou un mélange de ces composants.

15. Objet de l'une quelconque des revendications précédentes dans lequel l'ingrédient actif est un médicament et est éventuellement sélectionné parmi la nifédipine, le gemfibrozil, la terfénadine, le captopril, la cyclosporine, la calcitonine, les héparines et héparinoïdes, l'ingrédient actif étant par exemple la cyclosporine, ou l'ingrédient actif étant un agent thérapeutique sélectionné parmi les peptides, les protéines, les vaccins et les oligonucléotides.

16. Objet de l'une quelconque des revendications 1 à 14 dans lequel l'ingrédient actif est la nimodipine.
